(19) Europäisches Patentamt
European Patent Office
Office européen des brevets

(11) Publication number: 0 055 991 A2

# (12) EUROPEAN PATENT APPLICATION

(21) Application number: 82100885.1

(51) Int. Cl.$^3$: A 61 K 31/395

(22) Date of filing: 28.09.79

(30) Priority: 02.10.78 US 947374

(43) Date of publication of application: 14.07.82 Bulletin 82/28

(84) Designated Contracting States: AT BE CH DE FR GB IT LU NL SE

(60) Publication number of the earlier application in accordance with Art. 76 EPC: 0 009 944

(71) Applicant: MERCK & CO. INC.
126, East Lincoln Avenue P.O. Box 2000
Rahway, New Jersey 07065(US)

(72) Inventor: Firestone, Raymond A.
60 Hunter Avenue
Fanwood New Jersey 07023(US)

(74) Representative: Crampton, Keith John Allen et al,
D YOUNG & CO 10 Staple Inn
London WC1V 7RD(GB)

## (54) Lysosomotropic detergent therapeutic agents, compositions containing them and their uses.

(57) Compositions useful in the control of fertility, the inhibition of malignant cell growth, and the control of other diseases which accumulate macrophages at the disease site, comprising a pharmaceutical carrier and lysosomotropic substance comprising certain heterocyclic amines having at least one hydrophilic substituent and a pK in the range 4.9 to 9.

The compositions of the present invention are used to inhibit or alter the functions of lysosome-bearing cells.

EP 0 055 991 A2

Croydon Printing Company Ltd.

# LYSOSOMOTROPIC DETERGENT THERAPEUTIC AGENTS, COMPOSITIONS CONTAINING THEM AND THEIR USE

This invention relates to compositions useful in the control of fertility, in the inhibition of malignant cell growth, and in the control of other diseases which accumulate macrophages at the disease site.

Lysosomotropic substances have been designated as substances which are selectively taken up by lysosomes regardless of their chemical nature or mechanism of uptake. It has been suggested that the property of lysosomotropism is useful in the preparation of therapeutic agents having useful biological properties. These substances can enter the lysosomes preferentially and exert their biological influence because of their own unique biological activity. They also can be coupled with active drugs and, following preferential uptake by lysosomes, can be hydrolysed within the lysosome with release of the active drug at the target area. Thus, in one method of using the lysosomotropic agent DNA, a complex of DNA with the potent cytotoxic agent daunorubicin was formed. It was then found that the complex was preferentially taken up by the tumour cells and that the DNA was preferentially degraded by lysosomes leaving the active daunorubicin within the lysosome. A drawback of this particular method is that the toxic dose is very close to the therapeutic dose which makes the margin of safety very narrow.

The present invention is concerned with the inhibition of lysosome-bearing cells and involves the use of new compositions and new pharmaceutical formulations useful in inhibiting the function of lysosome-bearing cells.

Still more specifically, the present invention provides a pharmaceutical composition comprising a pharmaceutical carrier and a lysosomotropic substance having a pK of from 4.9 to 9 that is an alkyl-substituted heterocyclic amine of the formula :

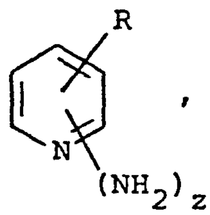 , 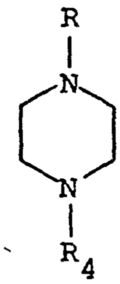 , 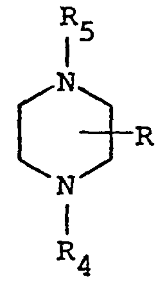 , 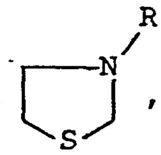 , 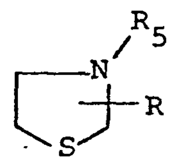 ,

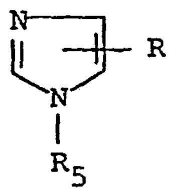 , , 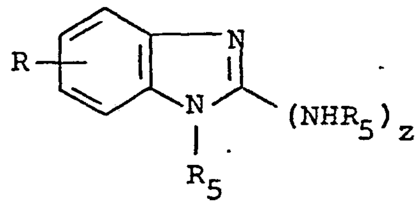 ,

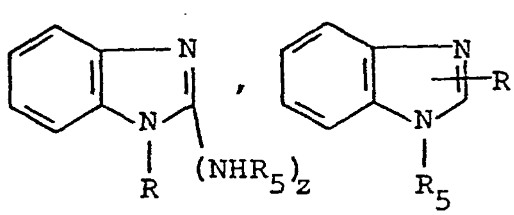 , 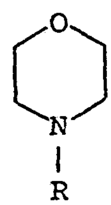 , or 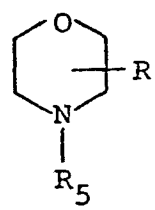

where

z is 0 or 1;

R is alkyl or unsaturated alkyl, each of which contains from 8-30 carbons, substituted alkyl of from 4-30 carbons, or a steroidal radical comprising 3-cholesteryl;

$R_4$ is acyl including $-COOR^1$, $-COR^1$ in which $R^1$ is alkyl or aryl, arylsulfonyl including

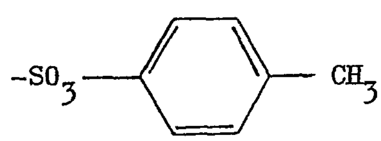 ,

or phosphoryl ($-PO_3$); and

$R_5$ is H or alkyl.

A preferred embodiment of the present invention is a pharmaceutical formulation comprising an inert carrier and an

alkyl-substituted nitrogen-containing heterocyclic compound of the formula :

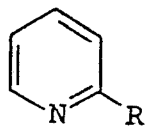 , 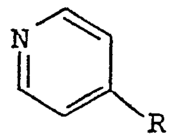 or 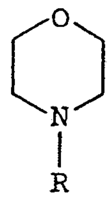 ,

where R is alkyl, alkenyl, or substituted alkyl of from 8 to 18 carbons.

The compositions of the present invention are useful in selectively killing or altering the function of lysosome-bearing cells, and they are also useful as systems for the delivery of an active drug to the disease site where the active drug is released by lysosomal enzymatic hydrolysis. Thus, the compositions of this invention are useful as antifertility agents. As antifertility agents, the active principle - for example, a compound of the formula :

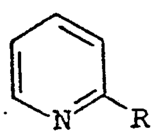 , 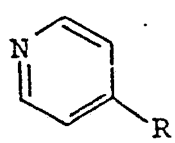 or 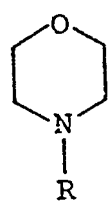 ,

in which R is as defined previously - is incorporated into vaginal or other contraceptive creams, jellies, or foams, e.g. in a concentration of from 0.1% to 10%, preferably 1-10%. The formulation prepared in this manner is administered intravaginally and contact of the sperm with the composition of the present invention incorporates the active principle into the acrosome of the sperm cells, thus rendering them infertile.

The compositions of the present invention are also useful in inhibiting the growth of malignant tumour cells. For this

purpose, the compositions of the present invention are administered either orally or by injection in a dosage schedule of 25-500 mg/kg/day.

The compositions of the present invention are also useful in combating infection caused by resistant microorganisms, which are sequestered within lysosomes and thus unavailable to attack by antibiotics. The lysosomotropic compositions of the present invention comprise an antibiotic incorporating a weakly basic amine group and a peptide linked to a carboxylic function of the antibiotic through an amide function. Such compositions are incorporated into the cell lysosome where they are hydrolysed by lysosomal enzymes, thus releasing the active antibiotic principle to attack the infecting microorganism.

Certain of the compositions of the present invention are useful in the treatment of inflammation. Thus cholesterol is rendered antiinflammatory by incorporating within it a lysosomotropic amine such as morpholine or trifluoroethylamine.

The compounds of this invention can be administered either topically or systemically (i.e., intravenously, subcutaneously, intramuscularly, orally, rectally, by aerosolization, or in the form of sterile implants for long action.

The pharmaceutical compositions can be sterile, injectable suspensions or solutions, or solid, orally administrable, pharmaceutically acceptable tablets or capsules; the compositions can also be intended for sub-lingual administration, or for suppository use. It is especially advantageous to formulate compositions in dosage unit forms for ease and economy of administration and uniformity of dosage. "Dosage unit form" as a term used herein refers to physically discrete units suitable as unitary dosages for animal and human subjects, each unit containing a predetermined quantity of active material calculated to produce the desired biological effect in association with required pharmaceutical means.

The low cost and ready accessibility of the compositions of this invention make them particularly promising for applications in veterinary medicine in which field their utilities are comparable to those in human medicine.

Examples of preferred heterocyclic compounds usable in the

compositions of the present invention are 4-alkyl pyridines such as 4-nonyl, 4-tridecyl, 4-heptadecyl, and 4-nonadecyl pyridine; 2-alkyl pyridines such as 2-nonyl, 2-tridecyl, 2-heptadecyl, and 2-nonadecyl pyridines; and N-alkyl morpholines such as N-decyl, N-dodecyl, N-tetradecyl, N-hexadecyl, and N-octadecyl morpholines.

The following Examples illustrate compositions in accordance with the present invention.

## EXAMPLE 1

### Contraceptive Formulations

A suitable formulation is prepared by mixing the lysosomotropic amine and other ingredients employed in the following proportions (in percentages):

| | |
|---|---|
| Benzyldimethyl [2-[2-(p-1,1,3,3-tetramethylbutylphenoxy)ethoxy]ethyl]ammonium chloride (benzethonium chloride, U.S.P.) | 0.2 |
| 2-nonylpyridine | 8.0 |
| Myristic acid | 2.0 |
| Stearic acid | 4.0 |
| Triethanolamine | 2.0 |
| Glyceryl monosterate | 3.0 |
| Polyoxyethylene (20) sorbitan mono-oleate | 3.0 |
| Polyoxyethylene (20) sorbitan monolaurate | 3.0 |
| Polyvinylpyrrolidone | 1.0 |
| Polyethylene glycol (average molecular weight, 600) | 1.4 |
| Deionized water | 72.4 |
| | 100.0 |

Other similar formulations are prepared by repeating the procedure using, in place of N-dodecyl-2,2,2-trifluoroethylamine, an identical amount of one of the following compounds: 4-alkyl pyridines such as 4-nonyl, 4-tridecyl, 4-heptadecyl, and 4-nonadecyl pyridine; other 2-alkyl pyridines such as 2-tridecyl, 2-heptadecyl, and 2-nonadecyl pyridines; and N-alkyl morpholines such as N-decyl, N-dodecyl, N-tetradecyl, N-hexadecyl, and N-octadecyl morpholines.

EXAMPLE 2

Capsule Formulation

| | |
|---|---|
| 2-nonylpyridine | 25 g. |
| Stearic Acid (U.S.P. triple pressure) | 125 g. |
| Pluronic F-68 | 7.5 g. |
| Corn Starch | 125 g. |

('Pluronic' is a registered trade mark)

The stearic acid and Pluronic are united in a vessel and melted using a water bath at 60-65°C. The heating is discontinued and the 2-nonylpyridine is dispersed into the mixture and the corn starch is added with stirring which is continued until the mixture cools to ambient temperature. The mixture is reduced to granules by screening and placed in a number 0 hard gelatin containing 282.5 mg. of total solids and 25 mg. of 2-nonylpyridine per capsule.

Other formulations are prepared by repeating the procedure using the same amount of one of the following heterocyclic compounds: 4-alkyl pyridines such as 4-nonyl, 4-tridecyl, 4-heptadecyl, and 4-nonadecyl pyridines; other 2-alkyl pyridines such as 2-tridecyl, 2-heptadecyl, and 2-nonadecyl pyridines; and N-alkyl morpholines such as N-decyl, N-dodecyl, N-tetradecyl, N-hexadecyl, and N-octadecyl morpholines.

CLAIMS

1. A pharmaceutical composition comprising a pharmaceutical carrier and a lysosomotropic amine compound having a pK of from 4.9 to 9 and that is an alkyl-substituted heterocyclic amine of the formula :

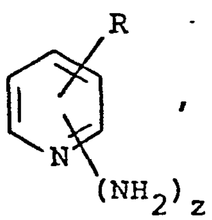 , 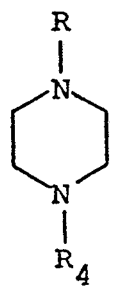 , 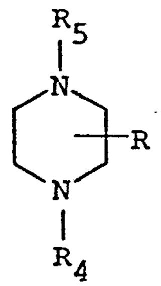 , 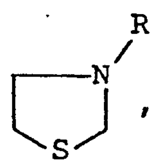 , 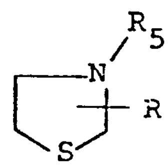 ,

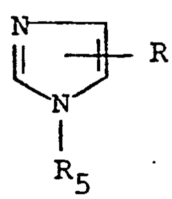 , , 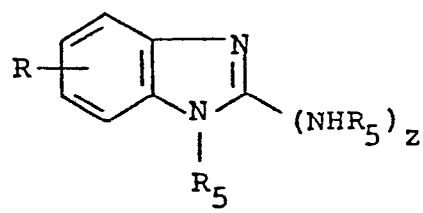 ,

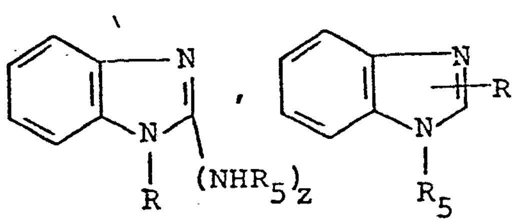 , 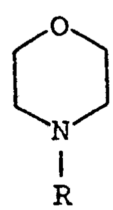 , or 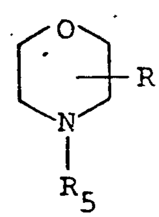

wherein

z is either 0 or 1;

R is alkyl or unsaturated alkyl, each of which contains from 8-30 carbons, substituted alkyl of from 4-30 carbons, or a steroidal radical comprising 3-cholesteryl;

$R_4$ is acyl including $-COOR^1m$ $-COR^1$ in which $R^1$ is alkyl or aryl, arylsulfonyl including

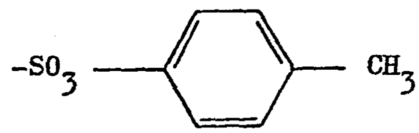

,

or phosphoryl $(-PO_3)$; and

$R_5$ is H or lower alkyl.

2. A composition according to Claim 1, in which the lysomotropic amine compound has the formula :

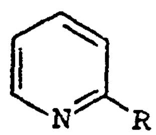

,

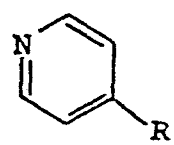

or

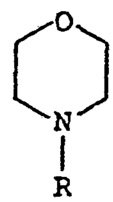

,

in which R is alkyl, alkenyl, or substituted alkyl of from 8 to 18 carbons.

3. A composition as claimed in Claim 1 or 2 for use in the inhibition or alteration of the function of lysosome-bearing cells.